(19) **Europäisches Patentamt**
**European Patent Office**
**Office européen des brevets**

(11) **EP 1 531 875 B1**

(12) **EUROPEAN PATENT SPECIFICATION**

(45) Date of publication and mention
of the grant of the patent:
**03.03.2010 Bulletin 2010/09**

(51) Int Cl.:
*A61L 27/16* (2006.01)    *A61L 27/54* (2006.01)
*A61L 31/16* (2006.01)    *A61L 31/04* (2006.01)

(21) Application number: **03785292.8**

(86) International application number:
**PCT/US2003/025510**

(22) Date of filing: **13.08.2003**

(87) International publication number:
**WO 2004/014447 (19.02.2004 Gazette 2004/08)**

(54) **ACTIVE AGENT DELIVERY SYSTEM INCLUDING A POLY(ETHYLENE-CO-(METH)ACRYLATE), MEDICAL DEVICE, AND METHOD**

DARREICHUNGSSYSTEME VON WIRKSTOFFEN MIT POLY(ETHYLENE-CO(METH)AKRYLATE, MEDIZINISCHE VORRICHTUNG UND VERFAHREN

SYSTEME D'ADMINISTRATION D'AGENT ACTIF COMPRENANT UN POLYETHYLENE-CO-METHACRYLATE, DISPOSITIF MEDICAL ET PROCEDE ASSOCIE

(84) Designated Contracting States:
**AT BE BG CH CY CZ DE DK EE ES FI FR GB GR HU IE IT LI LU MC NL PT RO SE SI SK TR**

(30) Priority: **13.08.2002 US 403413 P**

(43) Date of publication of application:
**25.05.2005 Bulletin 2005/21**

(73) Proprietor: **Medtronic, Inc.**
**Minneapolis, MN 55432-5604 (US)**

(72) Inventors:
• **LYU, SuPing**
**Maple Grove, MN 55311 (US)**
• **SPARER, Randall, V.**
**Andover, MN 55304 (US)**

• **HOBOT, Christopher, M.**
**Tonka Bay, MN 55331 (US)**
• **DANG, Kiem**
**Blaine, MN 55449 (US)**

(74) Representative: **Thomson, James B. et al**
**Dehns**
**St Bride's House**
**10 Salisbury Square**
**London**
**EC4Y 8JD (GB)**

(56) References cited:
**EP-A- 1 110 561        WO-A-03/022323**
**US-B1- 6 224 894**

**Description**

BACKGROUND OF THE INVENTION

A polymeric coating on a medical device may serve as a repository for delivery of an active agent (e.g., a therapeutic agent) to a subject. For many such applications, polymeric coatings must be as thin as possible. Polymeric materials for use in delivering an active agent may also be in various three-dimensional shapes.

Conventional active agent delivery systems suffer from limitations that include structural failure due to cracking and delamination from the device surface. Furthermore, they tend to be limited in terms of the range of active agents that can be used, the range of amounts of active agents that can be included within a delivery system, and the range of the rates at which the included active agents are delivered therefrom. This is frequently because many conventional systems include a single polymer.

Thus, there is a continuing need for active agent delivery systems with greater versatility and tunability.

SUMMARY OF THE INVENTION

The present invention provides active agent delivery systems that have generally good versatility and tunability in controlling the delivery of active agents as described in claim 1. Typically, such advantages result from the use of a blend of two or more miscible polymers. These delivery systems can be incorporated into medical devices, e.g., stents, stent grafts, anastomotic connectors, if desired.

The active agent delivery systems of the present invention typically include a blend of at least two miscible polymers, wherein at least one polymer (preferably one of the miscible polymers) is matched to the solubility of the active agent such that the delivery of the active agent preferably occurs predominantly under permeation control. In this context, "predominantly" with respect to permeation control means that at least 50%, preferably at least 75%, and more preferably at least 90%, of the total active agent load is delivered by permeation control.

Permeation control is typically important in delivering an active agent from systems in which the active agent passes through a miscible polymer blend having a "critical" dimension on a micron-scale level (i.e., the net diffusion path is no greater than about 1000 micrometers, although for shaped objects it can be up to about 10,000 microns). Furthermore, it is generally desirable to select polymers for a particular active agent that provide desirable mechanical properties without being detrimentally affected by nonuniform incorporation of the active agent.

The present invention provides an active agent delivery system that includes an active agent and a miscible polymer blend that includes a poly(ethylene-co-(meth)acrylate) (such as poly(ethylene-co-methyl acrylate)) and a second polymer not including poly(ethylene-co-vinyl acetate). The present invention provides an active agent delivery system that includes an active agent and a miscible polymer blend that includes the poly(ethylene-co-(meth)acrylate) and a second polymer not including poly(ethylene-co-vinyl acetate); the active agent that is hydrophobic and has a molecular weight of no greater than (i.e., less than or equal to) about 1200 grams per mole (g/mol); each of the active agent, the poly(ethylene-co-(meth)acrylate), and the second polymer has a solubility parameter; the difference between the solubility parameter of the active agent and the solubility parameter of the poly(ethylene-co-(meth)acrylate) is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$), and the difference between the solubility parameter of the active agent and at least one solubility parameter of the second polymer is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$); and the difference between the solubility parameter of the poly(ethylene-co-(meth)acrylate) and at least one solubility parameter of the second polymer is no greater than about 5 $J^{1/2}/cm^{3/2}$ (preferably, no greater than about 3 $J^{1/2}/cm^{3/2}$).

The present invention also provides medical devices that include such active agent delivery systems as described in claim 24.

A medical device is provided that includes: a substrate surface; a polymeric undercoat layer adhered to the substrate surface; and a polymeric top coat layer adhered to the polymeric undercoat layer; wherein the polymeric top coat layer includes an active agent incorporated within a miscible polymer blend that includes a a poly(ethylene-co-(meth) acrylate) and a second polymer not including poly(ethylene-co-vinyl acetate).

In another preferred embodiment, a stent is provided that includes: a substrate surface; a polymeric undercoat layer adhered to the substrate surface; and a polymeric top coat layer adhered to the undercoat layer; wherein the polymeric top coat layer includes an active agent incorporated within a miscible polymer blend that includes a poly (ethylene-co-(meth)acrylate) and a second polymer not including poly(ethylene-co-vinyl acetate).

The present invention also provides methods for making an active agent delivery system as described in claim 31.

A method of forming an active agent delivery system includes: combining a poly(ethylene-co-(meth)acrylate) and a second polymer not including poly(ethylene-co-vinyl acetate) to form a miscible polymer blend; and combining an active agent with the miscible polymer blend.

BRIEF DESCRIPTION OF THE DRAWINGS

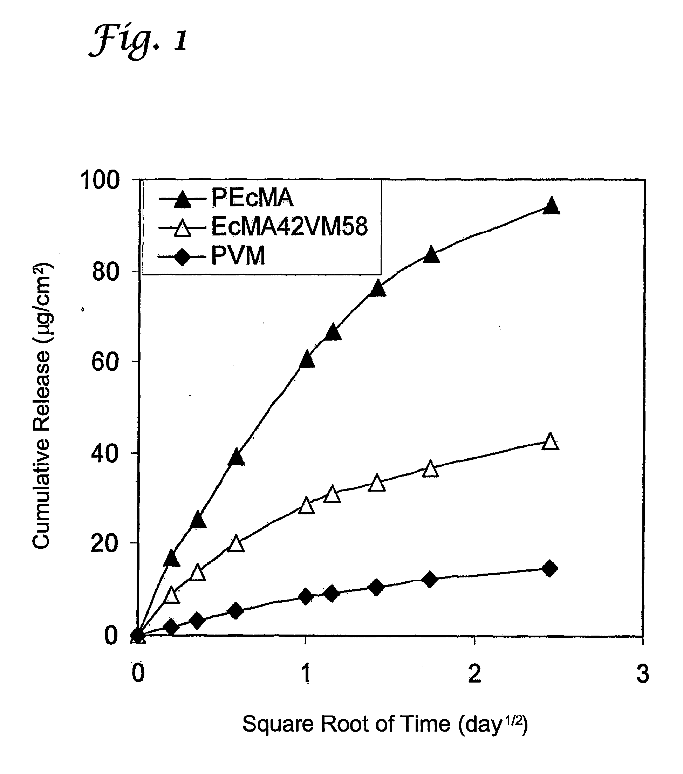

Figure 1. Graph showing the release profile of dexamethasone from a blend of 42 wt-% poly (ethylene-co-methyl acrylate) (PEcMA) and 58% poly (vinyl formal) (PVM). The release rate of dexamethasone from the polymer blend was between the rates of each of the unblended polymers, which demonstrates tunability of the blend system. The cumulative release amount was proportional to the square root of time, which demonstrates delivery by permeation control.

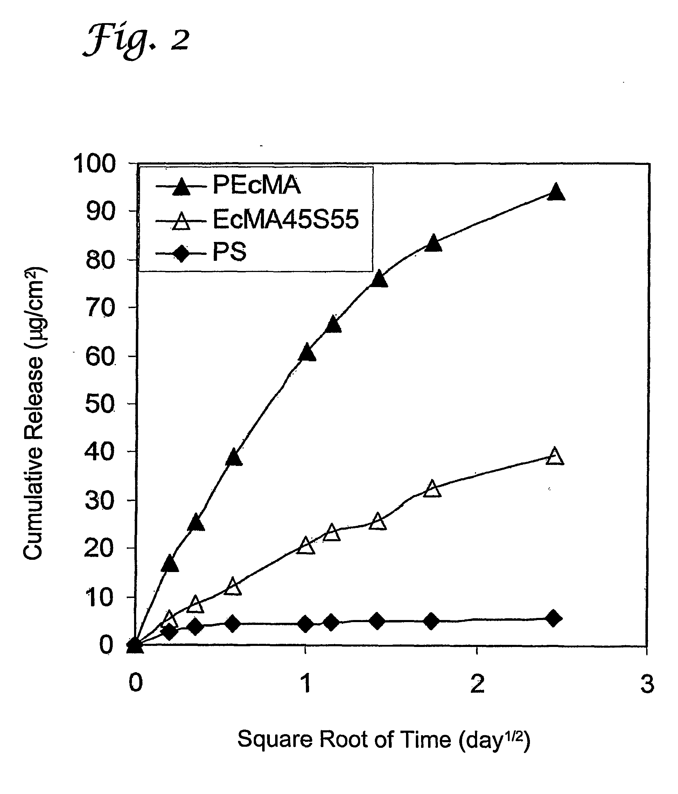

Figure 2. Graph showing the release profile of dexamethasone from a blend of 45 wt-% poly (ethylene-co-methyl acrylate) (PEcMA) and 55 wt-% polystyrene. The release rate of dexamethasone from the polymer blend was between the rates of each of the unblended polymers, which demonstrates tunability of the blend system. The cumulative release amount was proportional to square root of time, which demonstrates delivery by permeation control.

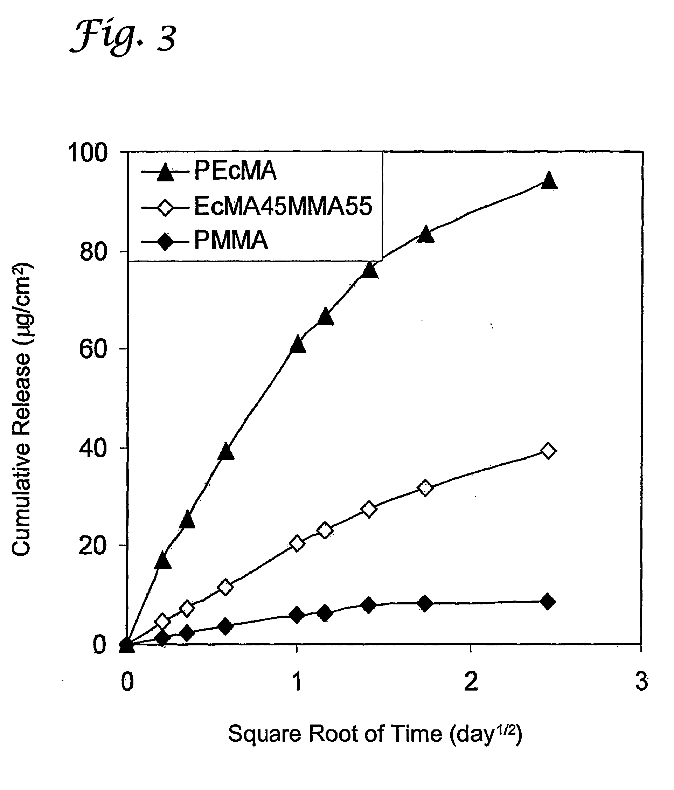

Figure 3. Graph showing the release profile of dexamethasone from a blend of 45 wt-% poly (ethylene-co-methyl acrylate) (PEcMA) and 55 wt-% poly(methyl methacrylate). The release rate of dexamethasone from the polymer blend was between the rates of each of the unblended polymers, which demonstrates tunability of the blend system. The cumulative release amount was proportional to square root of time, which demonstrates delivery by permeation control.

DETAILED DESCRIPTION OF ILLUSTRATIVE EMBODIMENTS

The present invention provides active agent delivery systems that include an active agent for delivery to a subject and a miscible polymer blend. The delivery systems can include a variety of polymers as long as at least two are miscible as defined herein. The active agent may be incorporated within the miscible polymer blend such that it is dissoluted from the blend, or the blend can initially function as a barrier to the environment through which the active agent passes.

Miscible polymer blends are advantageous because they can provide greater versatility and tunability for a greater range of active agents than can conventional systems that include immiscible mixtures or only a single polymer, for example. That is, using two or more polymers, at least two of which are miscible, can generally provide a more versatile active agent delivery system than a delivery system with only one of the polymers. A greater range of types of active agents can typically be used. A greater range of amounts of an active agent can typically be incorporated into and delivered from (preferably, predominantly under permeation control) the delivery systems of the present invention. A greater range of delivery rates for an active agent can typically be provided by the delivery systems of the present invention. At least in part, this is because of the use of a miscible polymer blend that includes at least two miscible polymers. It should be understood that, although the description herein refers to two polymers, the invention encompasses systems that include more than two polymers, as long as a miscible polymer blend is formed that includes at least two miscible polymers.

A miscible polymer blend of the present invention has a sufficient amount of at least two miscible polymers to form a continuous portion, which helps tune the rate of release of the active agent. Such a continuous portion (i.e., continuous phase) can be identified microscopically or by selective solvent etching. Preferably, the at least two miscible polymers form at least 50 percent by volume of a miscible polymer blend.

A miscible polymer blend as used herein can also optionally include a dispersed (i.e., discontinuous) immiscible portion. If both continuous and dispersed portions are present, the active agent can be incorporated within either portion. Preferably, the active agent is loaded into the continuous portion to provide delivery of the active agent predominantly under permeation control. To load the active agent, the solubility parameters of the active agent and the portion of the miscible polymer blend a majority of the active agent is loaded into are matched (typically to within no greater than about 10 $J^{1/2}/cm^{3/2}$, preferably, no greater than about 5 $J^{1/2}/cm^{3/2}$, and more preferably, no greater than about 3 $J^{1/2}/cm^{3/2}$). The continuous phase controls the release of the active agent regardless of where the active agent is loaded.

A miscible polymer blend, as used herein, encompasses a number of completely miscible blends of two or more polymers as well as partially miscible blends of two or more polymers. A completely miscible polymer blend will ideally have a single glass transition temperature (Tg) due to mixing at the molecular level over the entire concentration range. Partially miscible polymer blends may have multiple Tg's because mixing at the molecular level is limited to only parts of the entire concentration range. These partially miscible blends are included within the scope of the term "miscible polymer blend" as long as the absolute value of the difference between at least one Tg ($Tg_{polymer1}-Tg_{polymer2}$) for each of at least two polymers within the blend is reduced by the act of blending. Tg's can be determined by measuring the mechanical properties, thermal properties, electric properties, etc. as a function of temperature.

A miscible polymer blend can also be determined based on its optical properties. A completely miscible blend forms a stable and homogeneous domain that is transparent, whereas an immiscible blend forms a heterogeneous domain that scatters light and visually appears turbid unless the components have identical refractive indices. Additionally, a phase-separated structure of immiscible blends can be directly observed with microscopy. A simple method used in the present invention to check the miscibility involves mixing the polymers and forming a thin film of about 10 micrometers to about 50 micrometers thick. If such a film is generally as clear and transparent as the least clear and transparent film of the same thickness of the individual polymers prior to blending, then the polymers are completely miscible.

Miscibility between polymers depends on the interactions between them and their molecular structures and molecular weights. The interaction between polymers can be characterized by the so-called Flory-Huggins parameter ($\chi$). When $\chi$ is close to zero (0) or even is negative, the polymers are very likely miscible. Theoretically, $\chi$ can be estimated from the solubility parameters of the polymers, i.e., $\chi$ is proportional to the squared difference between them. Therefore, the miscibility of polymers can be approximately predicted. For example, the closer the solubility parameters of the two polymers are the higher the possibility that the two polymers are miscible. Miscibility between polymers tends to decrease as their molecular weights increases.

Thus, in addition to the experimental determinations, the miscibility between polymers can be predicted simply based on the Flory-Huggins interaction parameters, or even more simply, based the solubility parameters of the components. However, because of the molecular weight effect, close solubility parameters do not necessarily guarantee miscibility.

It should be understood that a mixture of polymers needs only to meet one of the definitions provided herein to be miscible. Furthermore, a mixture of polymers may become a miscible blend upon incorporation of an active agent. The types and amounts of polymers and active agents are typically selected to form a system having a preselected dissolution time (or rate) through a preselected critical dimension of the miscible polymer blend. Glass transition temperatures and solubility parameters can be used in guiding one of skill in the art to select an appropriate combination of components in an active agent delivery system, whether the active agent is incorporated into the miscible polymer blend or not. Solubility parameters are generally useful for determining miscibility of the polymers and matching the solubility of the active agent to that of the miscible polymer blend. Glass transition temperatures are generally useful for determining miscibility of the polymers and tuning the dissolution time (or rate) of the active agent. These concepts are discussed in greater detail below.

A miscible polymer blend can be used in combination with an active agent in the delivery systems of the present invention in a variety of formats as long as the miscible polymer blend controls the delivery of the active agent.

In one embodiment, a miscible polymer blend has an active agent incorporated therein. Preferably, such an active agent is dissoluted predominantly under permeation control, which requires at least some solubility of the active agent in the continuous portion (i.e., the miscible portion) of the polymer blend, whether the majority of the active agent is loaded in the continuous portion or not. Dispersions are acceptable as long as little or no porosity channeling occurs during dissolution of the active agent and the size of the dispersed domains is much smaller than the critical dimension of the blends, and the physical properties are generally uniform throughout the composition for desirable mechanical performance. This embodiment is often referred to as a "matrix" system.

In another embodiment, a miscible polymer blend initially provides a barrier to permeation of an active agent. This embodiment is often referred to as a "reservoir" system. A reservoir system can be in many formats with two or more layers. For example, a miscible polymer blend can form an outer layer over an inner layer of another material (referred to herein as the inner matrix material). In another example, a reservoir system can be in the form of a core-shell, wherein the miscible polymer blend forms the shell around the core matrix (i.e., the inner matrix material). At least initially upon formation, the miscible polymer blend in the shell or outer layer could be substantially free of active agent. Subsequently, the active agent permeates from the inner matrix and through the miscible polymer blend for delivery to the subject. The inner matrix material can include a wide variety of conventional materials used in the delivery of active agents. These include, for example, an organic polymer such as those described herein for use in the miscible polymer blends, or a wax, or a different miscible polymer blend. Alternatively, the inner matrix material can be the active agent itself.

For a reservoir system, the release rate of the active agent can be tuned with selection of the material of the outer layer. The inner matrix can include an immiscible mixture of polymers or it can be a homopolymer if the outer layer is a miscible blend of polymers.

As with matrix systems, an active agent in a reservoir system is preferably dissoluted predominantly under permeation control through the miscible polymer blend of the barrier layer (i.e., the barrier polymer blend), which requires at least some solubility of the active agent in the barrier polymer blend. Again, dispersions are acceptable as long as little or no porosity channeling occurs in the barrier polymer blend during dissolution of the active agent and the size of the dispersed domains is much smaller than the critical dimension of the blends, and the physical properties are generally uniform throughout the barrier polymer blend for desirable mechanical performance. Although these considerations may also be desirable for the inner matrix, they are not necessary requirements.

Typically, the amount of active agent within an active agent delivery system of the present invention is determined by the amount to be delivered and the time period over which it is to be delivered. Other factors can also contribute to the level of active agent present, including, for example, the ability of the composition to form a uniform film on a substrate.

Preferably, for a matrix system, an active agent is present within (i.e., incorporated within) a miscible polymer blend in an amount of at least about 0.1 weight percent (wt-%), more preferably, at least about 1 wt-%, and even more preferably, at least about 5 wt-%, based on the total weight of the miscible polymer blend and the active agent. Preferably, for a matrix system, an active agent is present within a miscible polymer blend in an amount of no greater than about 80 wt-%, more preferably, no greater than about 50 wt-%, and most preferably, no greater than about 30 wt-%, based on the total weight of the miscible polymer blend and the active agent. Typically and preferably, the amount of active agent will be at or below its solubility limit in the miscible polymer blend.

Preferably, for a reservoir system, an active agent is present within an inner matrix in an amount of at least about 0.1 wt-%, more preferably, at least about 10 wt-%, and even more preferably, at least about 25 wt-%, based on the total weight of the inner matrix (including the active agent). Preferably, for a reservoir system, an active agent is present within an inner matrix in an amount of up to 100 wt-%, and more preferably, no greater than about 80 wt-%, based on the total weight of the inner matrix (including the active agent).

In the active agent delivery systems of the present invention, an active agent is dissolutable through a miscible polymer blend. Dissolution is preferably controlled predominantly by permeation of the active agent through the miscible polymer blend. That is, the active agent initially dissolves into the miscible polymer blend and then diffuses through the miscible polymer blend predominantly under permeation control. Thus, as stated above, for certain preferred embodiments, the active agent is at or below the solubility limit of the miscible polymer blend. Although not wishing to be bound by theory, it is believed that because of this mechanism the active agent delivery systems of the present invention have a significant level of tunability.

If the active agent exceeds the solubility of the miscible polymer blend and the amount of insoluble active agent exceeds the percolation limit, then the active agent could be dissoluted predominantly through a porosity mechanism. In addition, if the largest dimension of the active agent insoluble phase (e.g., particles or aggregates of particles) is on the same order as the critical dimension of the miscible polymer blend, then the active agent could be dissoluted predominantly through a porosity mechanism. Dissolution by porosity control is typically undesirable because it does not provide effective predictability and controllability.

Because the active agent delivery systems of the present invention preferably have a critical dimension on the micron-scale level, it can be difficult to include a sufficient amount of active agent and avoid delivery by a porosity mechanism. Thus, the solubility parameters of the active agent and at least one polymer of the miscible polymer blend are matched to maximize the level of loading while decreasing the tendency for delivery by a porosity mechanism.

One can determine if there is a permeation-controlled release mechanism by examining a dissolution profile of the amount of active agent released versus time (t). For permeation-controlled release from a matrix system, the profile is directly proportional to $t^{1/2}$. For permeation-controlled release from a reservoir system, the profile is directly proportional to t. Alternatively, under sink conditions (i.e., conditions under which there are no rate-limiting barriers between the polymer blend and the media into which the active agent is dissoluted), porosity-controlled dissolution could result in a burst effect (i.e., an initial very rapid release of active agent).

The active agent delivery systems of the present invention, whether in the form of a matrix system or a reservoir system, for example, without limitation, can be in the form of coatings on substrates (e.g., open or closed cell foams, woven or nonwoven materials), films (which can be free-standing as in a patch, for example), shaped objects (e.g., microspheres, beads, rods, fibers, or other shaped objects), wound packing materials, etc.

As used herein, an "active agent" is one that produces a local or systemic effect in a subject (e.g., an animal). Typically, it is a pharmacologically active substance. The term is used to encompass any substance intended for use in the diagnosis, cure, mitigation, treatment, or prevention of disease or in the enhancement of desirable physical or mental development and conditions in a subject. The term "subject" used herein is taken to include humans, sheep, horses, cattle, pigs, dogs, cats, rats, mice, birds, reptiles, fish, insects, arachnids, protists (e.g., protozoa), and prokaryotic bacteria. Preferably, the subject is a human or other mammal.

Active agents can be synthetic or naturally occurring and include, without limitation, organic and inorganic chemical agents, polypeptides (which is used herein to encompass a polymer of L- or D- amino acids of any length including peptides, oligopeptides, proteins, enzymes, hormones, etc.), polynucleotides (which is used herein to encompass a polymer of nucleic acids of any length including oligonucleotides, single- and double-stranded DNA, single- and double-stranded RNA, DNA/RNA chimeras, etc.), saccharides (e.g., mono-, di-, poly-saccharides, and mucopolysaccharides), vitamins, viral agents, and other living material, radionuclides, and the like. Examples include antithrombogenic and anticoagulant agents such as heparin, coumadin, coumarin, protamine, and hirudin; antimicrobial agents such as antibiotics; antineoplastic agents and anti-proliferative agents such as etoposide, podophylotoxin; antiplatelet agents including aspirin and dipyridamole; antimitotics (cytotoxic agents) and antimetabolites such as methotrexate, colchicine, azathioprine, vincristine, vinblastine, fluorouracil, adriamycin, and mutamycinnucleic acids; antidiabetic such as rosiglitazone maleate; and anti-inflammatory agents. Anti-inflammatory agents for use in the present invention include glucocorticoids, their salts, and derivatives thereof, such as cortisol, cortisone, fludrocortisone, Prednisone, Prednisolone, 6α-methylprednisolone, triamcinolone, betamethasone, dexamethasone, beclomethasone, aclomethasone, amcinonide, clebethasol, and clocortolone. Preferably, the active agent is not heparin.

For preferred active agent delivery systems of the present invention, the active agent is typically matched to the solubility of the miscible portion of the polymer blend. For the present invention, at least one polymer of the polymer blend is hydrophobic. Thus, preferred active agents for the present invention are hydrophobic. Preferably, if the active agent is hydrophobic, then at least one of the miscible polymers is hydrophobic, and if the active agent is hydrophilic, then at least one of the miscible polymers is hydrophilic, although this is not necessarily required.

As used herein, in this context (in the context of the polymer of the blend), the term "hydrophobic" refers to a material that will not increase in volume by more than 10% or in weight by more than 10%, whichever comes first, when swollen by water at body temperature (i.e., about 37°C). In contrast, the term "hydrophilic" refers to a material that will increase in volume by at least 10% or in weight by at least 10%, whichever comes first, when swollen by water at body temperature (i.e., about 37°C).

As used herein, in this context (in the context of the active agent), the term "hydrophobic" refers to an active agent that has a solubility in water at room temperature (i.e., about 25°C) of no more than (i.e., less than or equal to) 200 micrograms per milliliter. In contrast, the term "hydrophilic" refers to an active agent that has a solubility in water of more than 200 micrograms per milliliter.

For delivery systems in which the active agent is hydrophobic, regardless of the molecular weight, polymers are typically selected such that the molar average solubility parameter of the miscible polymer blend is no greater than 28 $J^{1/2}/cm^{3/2}$ (preferably, no greater than 25 $J^{1/2}/cm^{3/2}$). For delivery systems in which the active agent is hydrophilic, regardless of the molecular weight, polymers are typically selected such that the molar average solubility parameter of the miscible polymer blend is greater than 21 $J^{1/2}/cm^{3/2}$ (preferably, greater than 25 $J^{1/2}/cm^{3/2}$). Herein "molar average solubility parameter" means the average of the solubility parameters of the blend components that are miscible with each other and that form the continuous portion of the miscible polymer blend.

These are weighted by their molar percentage in the blend, without the active agent incorporated into the polymer blend.

As the size of the active agent gets sufficiently large, diffusion through the polymer is affected. Thus, active agents can be categorized based on molecular weights and polymers can be selected depending on the range of molecular weights of the active agents.

For preferred active agent delivery systems of the present invention, the active agent has a molecular weight of no greater than about 1200 g/mol. For even more preferred embodiments, active agents of a molecular weight no greater than about 800 g/mol are desired.

Of the active agents listed above, those that are hydrophobic and have a molecular weight of no greater than about 1200 g/mol are particularly preferred.

As stated above, the types and amounts of polymers and active agents are typically selected to form a system having a preselected dissolution time (t) through a preselected critical dimension (x) of the miscible polymer blend. This involves selecting at least two polymers to provide a target diffusivity, which is directly proportional to the critical dimension squared divided by the time ($x^2/t$), for a given active agent.

The diffusivity can be easily measured by dissolution analysis using the following equation (see, for example, Kinam Park edited, Controlled Drug Delivery: Challenges and Strategies, American Chemical Society, Washington, DC, 1997):

$$D = (\frac{M_t}{4M_\infty})^2 \cdot \frac{\pi x^2}{t}$$

wherein D = diffusion coefficient; $M_t$ = cumulative release; $M_\infty$ = total loading of active agent; x = the critical dimension (e.g., thickness of the film); and t = the dissolution time. This equation is valid during dissolution of up to 60 percent by weight of the initial load of the active agent. Also, blend samples should be in the form of a film.

In refining the selection of the polymers for the desired active agent, the desired dissolution time (or rate), and the desired critical dimension, the parameters that can be considered when selecting the polymers for the desired active agent include glass transition temperatures of the polymers, solubility parameters of the polymers, and solubility parameters of the active agents. These can be used in guiding one of skill in the art to select an appropriate combination of components in an active agent delivery system, whether the active agent is incorporated into the miscible polymer blend or not.

For enhancing the tunability of a permeation-controlled delivery system, for example, preferably the polymers are selected such that the difference between at least one Tg of at least two of the polymers of the blend is sufficient to provide the target diffusivity. The target diffusivity is determined by the preselected dissolution time (t) for delivery and the preselected critical dimension (x) of the polymer composition and is directly proportional to $x^2/t$.

For enhancing the versatility of a permeation-controlled delivery system, for example, preferably the polymers are selected such that at least one of the following relationships is true: (1) the difference between the solubility parameter of the active agent and at least one solubility parameter of at least one polymer is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$); and (2) the difference between at least one solubility parameter of each at least two polymers is no greater than about 5 $J^{1/2}/cm^{3/2}$ (preferably, no greater than about 3 $J^{1/2}/cm^{3/2}$). More preferably, both relationships are true. Most preferably, both relationships are true for all polymers of the blend.

Typically, a compound has only one solubility parameter, although certain polymers, such as segmented copolymers and block copolymers, for example, can have more than one solubility parameter. Solubility parameters can be measured or they are calculated using an average of the values calculated using the Hoy Method and the Hoftyzer-van Krevelen Method (chemical group contribution methods), as disclosed in D.W. van Krevelen, Properties of Polymers, 3rd Edition, Elsevier, Amsterdam. To calculate these values, the volume of each chemical is needed, which can be calculated using the Fedors Method, disclosed in the same reference.

Solubility parameters can also be calculated with computer simulations, for example, molecular dynamics simulation and Monte Carlo simulation. Specifically, the molecular dynamics simulation can be conducted with Accelrys Materials Studio, Accelrys Inc., San Diego, CA. The computer simulations can be used to directly calculate the Flory-Huggins parameter.

A miscible polymer blend of the present invention includes a poly(ethylene-co-(meth)acrylate). Herein, a (meth)acrylate refers to both an acrylate and a methacrylate. A preferred poly(ethylene-co-(meth)acrylate) is poly(ethylene-co-methyl acrylate) (PEcMA). Poly(ethylene-co-methyl acrylate) (PEcMA) is preferably present in the miscible polymer blend in an amount of at least about 0.1 wt-%, and more preferably up to about 99.9 wt-%, based on the total weight of the blend, depending on the active agent and specific choice of polymers.

Preferably, higher molecular weights of polymers are desirable for better mechanical properties; however, the molecular weights should not be so high such that the polymer is not soluble in a processing solvent for preferred solvent-coating techniques or not miscible with the other polymer(s) in the blend. A preferred poly(ethylene-co-(meth)acrylate) has a number average molecular weight of at least about 10,000 g/mol, and more preferably at least about 20,000 g/mol. A preferred poly(ethylene-co-(meth)acrylate) has a number average molecular weight of no greater than about 200,000 g/mol, and more preferably no greater than about 100,000 g/mol, and most preferably no greater than about 70,000 g/mol.

A miscible polymer blend of the present invention includes a second polymer, not including poly(ethylene vinyl acetate), that is preferably present in the miscible polymer blend in an amount of at least about 0.1 wt-%, and more preferably up to about 99.9 wt-%, based on the total weight of the blend, depending on the active agent and specific choice of polymers.

The second polymer, not including poly(ethylene vinyl acetate), is preferably selected from the group consisting of a poly(vinyl alkylate), a poly(vinyl alkyl ether), a poly(vinyl acetal), a poly(alkyl and/or aryl methacrylate) or a poly(alkyl and/or aryl acrylate); and combinations thereof. In this context, "combinations" refers to mixtures and copolymers thereof. The mixtures and copolymers can include one or more members of the group and/or other monomers/polymers. Thus, polyvinyl copolymers include copolymers of vinyl alkylates, vinyl alkyl ethers, and vinyl acetals with each other and/or with a variety of other monomers including styrene, hydrogenated styrene, (meth)acrylates (i.e., esters of acrylic acid or methacrylic acid also referred to as acrylates and methacrylates, including alkyl and/or aryl (meth)acrylates), cyanoacrylates (i.e., esters of cyanoacrylic acid including alkyl and/or aryl cyanoacrylates), and acrylonitrile.

Preferred polyvinyl homopolymers or copolymers thereof include poly(vinyl formal), poly(vinyl butyral), poly(vinyl ether), poly(vinyl acetate), poly(vinyl propionate), poly(vinyl butyrate), and combinations thereof (i.e., mixtures and copolymers thereof). A particularly preferred polyvinyl homopolymer or copolymer is a homopolymer or copolymer of polyvinyl alkylates including, for example, poly(vinyl acetate), poly(vinyl propionate), or poly(vinyl butyrate). Of these, poly(vinyl acetate) is particularly desirable.

Preferred poly(alkyl methacrylate) polymers or poly(alkyl acrylate) (referred to generally as poly(alkyl (meth)acrylate) polymers or copolymers include poly(methyl methacrylate), poly(ethyl methacrylate), and poly(butyl methacrylate). Of these, poly(ethylene-co-ethyl acrylate) is particularly desirable.

Preferably, higher molecular weights of polymers are desirable for better mechanical properties; however, the molecular weights should not be so high such that the polymer is not soluble in a processing solvent for preferred solvent-coating techniques or not miscible with the other polymer(s) in the blend. A preferred hydrophobic second polymer has a number average molecular weight of at least about 10,000 g/mol, and more preferably at least about 50,000 g/mol. A preferred hydrophobic second polymer has a weight average molecular weight of no greater than about 1,000,000 g/mol, and more preferably no greater than about 200,000 g/mol.

Preferably, the second polymer has a higher glass transition temperature (Tg) than the poly(ethylene-co-methyl acrylate) (PEcMA). For example, a preferred combination includes polyvinyl butyral-co-vinyl alcohol-co-vinyl acetate, which has a Tg of 72-78°C, and poly(ethylene-co-methyl acrylate) (PEcMA), which has a Tg of 7°C. By combining such high and low Tg polymers, the active agent delivery system can be tuned for the desired dissolution time of the active agent.

Preferably, at least one of the following is true: the difference between the solubility parameter of the active agent and the solubility parameter of the poly(ethylene-co-(meth)acrylate) is no greater than about 10 $J^{1/2}/cm^{3/2}$ (preferably; no greater than about 5 $J^{1/2}/cm^{3/2}$, and more preferably, no greater than about 3 $J^{1/2}/cm^{3/2}$); and the difference between the solubility parameter of the active agent and at least one solubility parameter of the second is no greater than about 10 $J^{1/2}/cm^{3/2}$ (preferably, no greater than about 5 $J^{1/2}/cm^{3/2}$, and more preferably, no greater than about 3 $J^{1/2}/cm^{3/2}$). More preferably, both of these statements are true. Preferably, the difference between the solubility parameter of the poly(ethylene-co-(meth)acrylate) and the second polymer is no greater than about 5 $J^{1/2}/cm^{3/2}$ (preferably, no greater than about 3 $J^{1/2}/cm^{3/2}$).

Table 1: Tg and solubility parameters for polymers. All data are from the vendor except where indicated.

| Polymers | Tg (°C) | Solubility parameter ($J^{1/2}/cm^{3/2}$) | Notes | Sources |
|---|---|---|---|---|
| Poly (ethylene-co-methyl acrylate) (PEcMA) | 7 (DSC) | 16.9 [a] | d = 0.948 g/mL MA, 27 wt-% Mn = 13 kg/mol, Mw = 72.5 kg/mol | Sigma-Aldrich Co., Milwaukee, WI. Product No. 432660 |
| Poly (vinyl acetate) (PVAC) | 28 [b] | 20.9 [c] | Mw = 500 kg/mol | Sigma-Aldrich Co., Milwaukee, WI. Product No. 387932 |
| Poly (vinyl formal) (PVM) | 108 | 20.4 [d] | d = 1.23 g/mL | Sigma-Aldrich Co., Milwaukee, WI. Product No. 182680 |
| Poly (vinyl butyral-co-vinyl alcohol-co-vinyl acetate) (PVBVAVAC) | 72-78 | 23.1 [d,e] | Mw = 170-250 kg/mol, VB, VA, and VAC = 80, 17.5-20, and 0-2.5 wt-%. | Sigma-Aldrich Co., Milwaukee, WI. Product No. 418420 |
| Poly (styrene) (PS) | 95 | 18.2 [c] | d = 1.04 g/mL Mw = 350 kg/mol Mn=170 kg/mol | Sigma-Aldrich Co., Milwaukee, WI. Product No. 441147 |
| Poly (butyl methacrylate) (PBMA) | 15 | 18.1 [c] | d=1.07 g/mL, Mw = 337 kg/mol | Sigma-Aldrich Co., Milwaukee, WI. Product No. 181528 |
| Poly (methyl methacrylate) (PMMA) | 122 | 19.0 [d] 22.4 [c] | d = 1.17 g/mL Mw = 350 kg/mol | Sigma-Aldrich Co., Milwaukee, WI. Product No. 445746 |

(continued)

| Polymers | Tg (°C) | Solubility parameter ($J^{1/2}/cm^{3/2}$) | Notes | Sources |
|---|---|---|---|---|
| Poly (ethyl methacrylate) (PEMA) | 65 | 18.5 [c] | d = 1.16 g/mL Mw = 850 kg/mol | Sigma-Aldrich Co., Milwaukee, WI. Product No. 445789 |
| a. Average of polyethylene (PE) and poly (methyl acrylate) (PMA) weighted by their molar percentages. The solubility parameters of PE and PMA were from D.W.van Krevelen, Properties of Polymers, 3rd ed., Elsevier, 1990. Table 7.5. Data were the average if there were two values listed in the sources. b. Table 6.6, M. J. He, W. X. Chen, and X. X. Dong, Polymer Physics, revised version, FuDan University Press, ShangHai, China, 2000. Data were the average if there were two values listed in the sources. c. D.W.van Krevelen, Properties of Polymers, 3rd ed., Elsevier, 1990. Table 7.5. Data were the average if there were two values listed in the sources. d. The average of the calculated values based on Hoftyzer and van Kevelen's (H-vK) method (where the volumes of the chemicals were calculated based on Fedors' method) and Hoy's method. See Chapter 7, D.W.van Krevelen, Properties of Polymers, 3rd ed., Elsevier,1990, for details of all the calculations, where Table 7.8 was for Hoftyzer and van Kevelen's method, Table 7.3 for Fedors' method, and Table 7.9 and 7.10 for Hoy's method. e. The solubility parameter of the VBVAVAC was an average mased on the molar percentages of the VB, VA, and VAC. | | | | |

The polymers in the miscible polymer blends can be crosslinked or not. Similarly, the blended polymers can be crosslinked or not. Such crosslinking can be carried out by one of skill in the art after blending using standard techniques.

In the active agent systems of the present invention, the active agent passes through a miscible polymer blend having a "critical" dimension. This critical dimension is along the net diffusion path of the active agent and is preferably no greater than about 1000 micrometers (i.e., microns), although for shaped objects it can be up to about 10,000 microns.

For embodiments in which the miscible polymer blends form coatings or free-standing films (both generically referred to herein as "films"), the critical dimension is the thickness of the film and is preferably no greater than about 1000 microns, more preferably no greater than about 500 microns, and most preferably no greater than about 100 microns. A film can be as thin as desired (e.g., 1 nanometer), but are preferably no thinner than about 10 nanometers, more preferably no thinner than about 100 nanometers. Generally, the minimum film thickness is determined by the volume that is needed to hold the required dose of active agent and is typically only limited by the process used to form the materials. For all embodiments herein, the thickness of the film does not have to be constant or uniform. Furthermore, the thickness of the film can be used to tune the duration of time over which the active agent is released.

For embodiments in which the miscible polymer blends form shaped objects (e.g., microspheres, beads, rods, fibers, or other shaped objects), the critical dimension of the object (e.g., the diameter of a microsphere or a rod) is preferably no greater than about 10,000 microns, more preferably no greater than about 1000 microns, even more preferably no greater than about 500 microns, and most preferably no greater than about 100 microns. The objects can be as small as desired (e.g., 10 nanometers for the critical dimension). Preferably, the critical dimension is no less than about 100 microns, and more preferably no less than about 500 nanometers.

In one embodiment, the present invention provides a medical device characterized by a substrate surface overlayed with a polymeric top coat layer that includes a miscible polymer blend, preferably with a polymeric undercoat (primer) layer. When the device is in use, the miscible polymer blend is in contact with a bodily fluid, organ, or tissue of a subject.

The invention is not limited by the nature of the medical device; rather, any medical device can include the polymeric coating layer that includes the miscible polymer blend. Thus, as used herein, the term "medical device" refers generally to any device that has surfaces that can, in the ordinary course of their use and operation, contact bodily tissue, organs or fluids such as blood. Examples of medical devices include, without limitation, stents, stent grafts, anastomotic connectors, leads, needles, guide wires, catheters, sensors, surgical instruments, angioplasty balloons, wound drains, shunts, tubing, urethral inserts, pellets, implants, pumps, vascular grafts, valves, pacemakers, and the like. A medical device can be an extracorporeal device, such as a device used during surgery, which includes, for example, a blood oxygenator, blood pump, blood sensor, or tubing used to carry blood, and the like, which contact blood which is then returned to the subject. A medical device can likewise be an implantable device such as a vascular graft, stent, stent graft, anastomotic connector, electrical stimulation lead, heart valve, orthopedic device, catheter, shunt, sensor, replacement device for nucleus pulposus, cochlear or middle ear implant, intraocular lens, and the like. Implantable devices include transcutaneous devices such as drug injection ports and the like.

In general, preferred materials used to fabricate the medical device of the invention are biomaterials. A "biomaterial" is a material that is intended for implantation in the human body and/or contact with bodily fluids, tissues, organs and the like, and that has the physical properties such as strength, elasticity, permeability and flexibility required to function for the intended purpose. For implantable devices in particular, the materials used are preferably biocompatible materials, i.e., materials that are not overly toxic to cells or tissue and do not cause undue harm to the body. The invention is not limited by the nature of the substrate surface for embodiments in which the miscible polymer blends form polymeric coatings. For example, the substrate surface can be composed of ceramic, glass, metal, polymer, or any combination thereof. In embodiments having a metal substrate surface, the metal is typically iron, nickel, gold, cobalt, copper, chrome, molybdenum, titanium, tantalum, aluminum, silver, platinum, carbon, and alloys thereof. A preferred metal is stainless steel, a nickel titanium alloy, such as NITINOL, or a cobalt chrome alloy, such as NP35N.

A polymeric coating that includes a miscible polymer blend can adhere to a substrate surface by either covalent or non-covalent interactions. Non-covalent interactions include ionic interactions, hydrogen bonding, dipole interactions, hydrophobic interactions and van der Waals interactions, for example.

Preferably; the substrate surface is not activated or functionalized prior to application of the miscible polymer blend coating, although in some embodiments pretreatment of the substrate surface may be desirable to promote adhesion. For example, a polymeric undercoat layer (i.e., primer) can be used to enhance adhesion of the polymeric coating to the substrate surface. Suitable polymeric undercoat layers are disclosed in Applicants, application US 2004/0 039 437 filed on August 13, 2002 and entitled MEDICAL DEVICE EXHIBITING IMPROVED ADHESION BETWEEN POLYMERIC COATING AND SUBSTRATE. A particularly preferred undercoat layer disclosed therein consists essentially of a polyurethane. Such a preferred undercoat layer includes a polymer blend that contains polymers other than polyurethane but only in amounts so small that they do not appreciably affect the durometer, durability, adhesive properties, structural integrity and elasticity of the undercoat layer compared to an undercoat layer that is exclusively polyurethane.

When a stent or other vascular prosthesis is implanted into a subject, restenosis is often observed during the period beginning shortly after injury to about four to six months later. Thus, for embodiments of the invention that include stents, the generalized dissolution rates contemplated are such that the active agent should ideally start to be released immediately after the prosthesis is secured to the lumen wall to lessen cell proliferation. The active agent should then continue to dissolute for up to about four to six months in total.

The invention is not limited by the process used to apply the polymer blends to a substrate surface to form a coating. Examples of suitable coating processes include solution processes, powder coating, melt extrusion, or vapor deposition.

A preferred method is solution coating. For solution coating processes, examples of solution processes include spray coating, dip coating, and spin coating. Typical solvents for use in a solution process include tetrahydrofuran (THF), methanol, ethanol, ethylacetate, dimethylformamide (DMF), dimethyacetamide (DMA), dimethylsulfoxide (DMSO), dioxane, N-methyl pyrollidone, chloroform, hexane, heptane, cyclohexane, toluene, formic acid, acetic acid, and/or dichloromethane. Single coats or multiple thin coats can be applied.

Similarly, the invention is not limited by the process used to form the miscible polymer blends into shaped objects. Such methods would depend on the type of shaped object. Examples of suitable processes include extrusion, molding, micromachining, emulsion polymerization methods, electrospray methods, etc.

For preferred embodiments in which the active agent delivery system includes one or more coating layers applied to a substrate surface, a preferred embodiment includes the use of a primer, which is preferably applied using a "reflow method," which is described in Applicants' application US 2004/0 039 437 filed on August 13, 2002 and entitled MEDICAL DEVICE EXHIBITING IMPROVED ADHESION BETWEEN POLYMERIC COATING AND SUBSTRATE. Preferably, in this "reflow method," the device fabrication process involves first applying an undercoat polymer to a substrate surface to form the polymeric undercoat layer, followed by treating the polymeric undercoat layer to reflow the undercoat polymer, followed by applying a miscible polymer blend, preferably with an active agent incorporated therein, to the reformed undercoat layer to form a polymeric top coat layer. Reflow of the undercoat polymer can be accomplished in any convenient manner, e.g., thermal treatment, infrared treatment, ultraviolet treatment, microwave treatment, RF treatment, mechanical compression, or solvent treatment. To reflow the undercoat polymer, the undercoat layer is heated to a temperature that is at least as high as the "melt flow temperature" of the undercoat polymer, and for a time sufficient to reflow the polymer. The temperature at which the polymer enters the liquid flow state (i.e., the "melt flow temperature") is the preferred minimum temperature that is used to reflow the polymer according to the invention. Typically 1 to 10 minutes is the time period used to reflow the polymer using a thermal treatment in accordance with the invention. The melt flow temperature for a polymer is typically above the Tg (the melt temperature for a glass) and the Tm (the melt temperature of a crystal) of the polymer.

EXAMPLES

The present invention is illustrated by the following examples. It is to be understood that the particular examples, materials, amounts, and procedures are to be interpreted broadly in accordance with the scope and spirit of the invention as set forth herein.

Example 1

Poly(ethylene-co-methyl acrylate) (PEcMA)/Poly (vinyl formal) (PVM) with Dexamethasone (DX)

PEcMA and PVM were used in this example to control the release of dexamethasone (DX). The glass transition temperature, solubility parameter, molecular weight, vendor information for each of the polymers are listed in Table 1. As the difference in the solubility parameters of the two polymers was about 3.5 $J^{1/2}/cm^{3/2}$, these two polymers were considered as miscible polymers as defined herein. Dexamethasone was also purchased from Sigma-Aldrich Co., Milwaukee, WI. The two polymers were dried at room temperature under reduced pressure overnight, and then were individually dissolved with anhydrous tetrahydrofuran (THF) (Sigma-Aldrich) to make 4 wt-% to 5 wt-% solutions. DX was dissolved using the same THF to make a solution of about 0.141 wt-%. The three solutions were mixed in different amounts to make three blend solutions that contained about 0 wt-%, 40 wt-%, and 100 wt-% PEcMA, based on the total weight of solids. Each solution contained about 10 wt-% DX, based on the total weight of solids. The blend solutions were coated on the surfaces of stainless steel (316L) shims of about 1.27 cm by 3.81 cm, which had previously been rinsed with THF and dried. The coated shims were stored under nitrogen gas at room temperature overnight to remove the solvent. The shims were weighed after each step of the experiment. Based on the weight differences, the total amount of drug/polymer coating was determined for each shim as was the thickness of the coating. In this example, the typical weight of the dried coating was about 4 milligrams (mg) to 10 mg per shim and the thickness was about 10 micrometers (microns) to 20 microns.

Dissolution of drug from PEcMA/PVM polymer matrix was conducted with the polymer/drug coated shims prepared above. The coated shims were cut into pieces that contained about 2 mg of coating. Each piece was immersed in a vial containing 3 milliliters (mL) of phosphate buffered saline solution (PBS, potassium phosphate monobasic (NF tested), 0.144 g/L, sodium chloride (USP tested), 9 g/L, and sodium phosphate dibasic (USP tested), 0.795 g/L, pH = 7.0 to 7.2 at 37°C, purchased from HyClone, Logan UT) that was preheated to 37°C. The dissolution test was run at 37°C and the samples were agitated on a shaker at about 10 revolutions per minute (rpm). The samples were analyzed at various times to determine the concentration of drug in the sample by collecting the PBS. After each collection, the PBS was refreshed. The concentration of DX in PBS was measured with UV-Vis spectroscopy (HP 4152A) at the wavelength of 243 nm. The concentration of DX in each sample was calculated by comparing to a standard curve created with a series of solutions of known concentrations.

Dissolution Data Analysis

Cumulative release of dexamethasone from the PEcMA/PVM blend matrix was plotted in Figure 1. The release rate of dexamethasone from PEcMA was much faster than that from PVM. The release rate for the miscible polymer blend was between that of the unblended polymers. These release curves clearly show that the release rate can be tuned by using a miscible polymer blend and adjusting the ratio of polymers in the blend. The cumulate release from all three matrices was almost linear with the square root of time, which indicates that there was no burst and the delivery of DX was under permeation control.

Example 2

Poly(ethylene-co-methyl acrylate) (PEcMA)/Polystyrene (PS) with Dexamethasone (DX)

PEcMA and PS were used in this example to control the release of DX. The glass transition temperature, solubility parameter, molecular weight, vendor information for each of the polymers are listed in Table 1. As the difference in the solubility parameters of the two polymers was about 1.3 $J^{1/2}/cm^{3/2}$, these two polymers were considered to be miscible polymers as defined herein. Dexamethasone was the same as that used in Example 1. Sample preparation, dissolution, and data analysis were the same as in Example 1. The release curves are shown in Figure 2. The release rate of dexamethasone was slower from PVM than from PEcMA. The release rate of DX from the miscible blend of PS and PEcMA was in between the rates of the unblended polymers. These release curves clearly show that the release rate can be tuned using a miscible polymer blend. The cumulative release of DX was proportional to the square root of time (no burst was observed) suggesting the delivery of DX from PEcMA/PS blends was under permeation control.

Example 3

Poly(ethylene-co-methyl acrylate) (PEcMA)/Poly(methyl methacrylate) (PMMA) With Dexamethasone (DX)

PEcMA and PMMA were used in this example to control the release of DX. The glass transition temperature, solubility parameter, molecular weight, vendor information for each of the polymers are listed in Table 1. As the difference in the solubility parameters of the two polymers was about 2.1 $J^{1/2}/cm^{3/2}$, these two polymers were considered to be miscible polymers as defined herein. Dexamethasone was the same as that used in Example 1. Sample preparation, dissolution, and data analysis were the same as described in Example 1. As shown in Figure 3, the release rate of DX from PEcMA was much faster than from PMMA. The release rate of DX from the miscible blend of PMMA and PEcMA was in between the rates of the unblended polymers. These release curves clearly show that the release rate can be tuned using a miscible polymer blend. The cumulative release of DX is also proportional to the square root of time (no burst was observed) suggesting the delivery of DX from PEcMA/PMMA blends was under permeation control.

**Claims**

1.   An active agent delivery system comprising an active agent and a miscible polymer blend comprising a poly(ethylene-co-(meth)acrylate) and a second polymer not including poly(ethylene vinyl acetate), wherein the second polymer is a polyvinyl alkylate homopolymer or copolymer, a polyalkyl and/or aryl methacrylate or acrylate or copolymer, a polyvinyl acetal or copolymer or a poly(vinyl alkyl ether) or copolymer; and wherein:

   each of the active agent, the poly(ethylene-co-(meth)acrylate) and the second polymer has a solubility parameter; and at least one of the following relationships is true:
   the difference between the solubility parameter of the active agent and the solubility parameter of the poly (ethylene-co-(meth)acrylate) is no greater than about 10 $J^{1/2}/cm^{3/2}$; and
   the difference between the solubility parameter of the active agent and at least one solubility parameter of the second polymer is no greater than about 10 $J^{1/2}/cm^{3/2}$.

2.   The system of claim 1 wherein the active agent is incorporated within the miscible polymer blend.

3.   The system of claim 2 wherein the active agent is present within the miscible polymer blend in an amount of about 0.1 wt-% to about 80 wt-%, based on the total weight of the miscible polymer blend and the active agent.

4.   The system of claim 1 wherein the miscible polymer blend initially provides a barrier to permeation of the active agent.

5.   The system of claim 4 wherein the active agent is incorporated within an inner matrix.

6.   The system of claim 5 wherein the active agent is present within the inner matrix in an amount of about 0.1 wt-% to about 100 wt-%, based on the total weight of the inner matrix including the active agent.

7.   The system of claim 1 wherein the difference between the solubility parameter of the poly(ethylene-co-(meth)acrylate) and at least one solubility parameter of the second polymer is no greater than about 5 $J^{1/2}/cm^{3/2}$.

8.   The system of claim 1 wherein the second polymer is a polyvinyl alkylate homopolymer or copolymer.

9.   The system of claim 1 wherein the second polymer is a polyalkyl and/or aryl methacrylate or acrylate or copolymer.

10.  The system of claim 1 wherein the second polymer is a polyvinyl acetal or copolymer.

11.  The system of claim 1 wherein the active agent is hydrophobic and has a molecular weight of no greater than about 1200 g/mol.

12.  The system of claim 1 wherein the poly(ethylene-co-(meth)acrylate) is present in the miscible polymer blend in an amount of about 0.1 wt-% to about 99.9 wt-%, based on the total weight of the blend.

13.  The system of claim 1 wherein the second polymer is present in the miscible polymer blend in an amount of about 0.1 wt-% to about 99.9 wt-%, based on the total weight of the blend.

**14.** The system of claim 1 which is in the form of microspheres, beads, rods, fibres, or other shaped objects.

**15.** The system of claim 14 wherein the critical dimension of the object is no greater than about 10,000 microns.

**16.** The system of claim 1 which is in the form of a film.

**17.** The system of claim 16 wherein the thickness of the film is no greater than about 1000 microns.

**18.** The system of claim 16 wherein the film forms a patch or a coating on a surface.

**19.** The active agent delivery system of claim 1, wherein delivery of the active agent occurs predominantly under permeation control.

**20.** The active agent delivery system of claim 1 wherein:

the active agent is hydrophobic and has a molecular weight of no greater than about 1200 g/mol;
the difference between the solubility parameter of the active agent and the solubility parameter of the poly (ethylene-co-(meth)acrylate) is no greater than about 10 $J^{1/2}/cm^{3/2}$, and the difference between the solubility parameter of the active agent and at least one solubility parameter of the second polymer is no greater than about 10 $J^{1/2}/cm^{3/2}$; and
the difference between the solubility parameter of the poly(ethylene-co-(meth)acrylate) and at least one solubility parameter of the second polymer is no greater than about 5 $J^{1/2}/cm^{3/2}$.

**21.** A medical device comprising the active agent delivery system of claim 1.

**22.** A medical device comprising the active agent delivery system of claim 19.

**23.** A medical device comprising the active agent delivery system of claim 20.

**24.** A medical device comprising:

a substrate surface;
a polymeric undercoat layer adhered to the substrate surface; and
a polymeric top coat layer adhered to the polymeric undercoat layer;
wherein the polymeric top coat layer comprises the active agent delivery system of claim 1 or 11 in which the active agent is incorporated within the miscible polymer blend.

**25.** The medical device of claim 24 wherein the polymer undercoat layer comprises a polyurethane.

**26.** The medical device of claim 24 which is an implantable device.

**27.** The medical device of claim 24 which is an extracorporeal device.

**28.** The medical device of claim 24 selected from the group consisting of a stent, stent graft, anastomic connector, lead, needle, guide wire, catheter, sensor, surgical instrument, angioplasty balloon, wound drain, shunt, tubing, urethral insert, pellet, implant, blood oxygenator, pump, vascular graft, valve, pacemaker, orthopedic device, replacement device for nucleus pulposes, and intraocular lense.

**29.** The medical device of claim 24 wherein said device is a stent.

**30.** The medical device of claim 24 or claim 29 wherein delivery of the active agent occurs predominantly under permeation control.

**31.** A method of forming an active agent delivery system as claimed in claim 1 comprising:

combining a poly(ethylene-co-(meth)acrylate) and a second polymer as defined in claim 1 to form a miscible polymer blend; and
combining an active agent with the miscible polymer blend.

32. The method of claim 31 wherein the active agent is incorporated within the miscible polymer blend.

33. The method of claim 31 wherein the active agent is incorporated within an inner matrix and the miscible polymer blend initially provides a barrier to permeation of the active agent.

34. The method of claim 31 wherein the active agent is hydrophobic and has a molecular weight of no greater than about 1200 g/mol.

**Patentansprüche**

1. Abgabesystem für einen aktiven Wirkstoff mit einem aktiven Wirkstoff und einer Mischung mischbarer Polymere, die ein Ethylen-/(Meth-)Acrylat-Kopolymer bzw. ein Poly(ethylen-co-(meth)acrylat) und ein zweites Polymer, das nicht Polyethylen-Vinylacetat umfasst, aufweist, wobei das zweite Polymer ein Polyvinylalkylat-Homopolymer oder ein Kopolymer, ein Polyalkyl- und/oder Polyaryl-Methacrylat oder ein Acrylat oder ein Kopolymer, ein Polyvinyl-Acetal oder ein Kopolymer oder ein Polyvinylalkylether oder ein Kopolymer ist, und wobei:

   der aktive Wirkstoff, das Poly(ethylen-co-(meth)acrylat) und das zweite Polymer jeweils einen Löslichkeitsparameter aufweisen; und wenigstens eine der nachfolgenden Beziehungen zutrifft:
   der Unterschied zwischen dem Löslichkeitsparameter des aktiven Wirkstoffs und dem Löslichkeitsparameter des Poly(ethylen-co-(meth)acrylats) beträgt nicht mehr als etwa 10 $J^{1/2}/cm^{3/2}$; und
   der Unterschied zwischen dem Löslichkeitsparameter des aktiven Wirkstoffs und wenigstens einem Löslichkeitsparameter des zweiten Polymers beträgt nicht mehr als etwa 10 $J^{1/2}/cm^{3/2}$.

2. System nach Anspruch 1, wobei der aktive Wirkstoff in der Mischung mischbarer Polymere integriert bzw. enthalten ist.

3. System nach Anspruch 2, wobei der aktive Wirkstoff in der Mischung mischbarer Polymere in einer Menge von etwa 0,1 Gew.-% bis etwa 80 Gew.-%, bezogen auf das Gesamtgewicht der Mischung mischbarer Polymere und des aktiven Wirkstoffs, vorliegt.

4. System nach Anspruch 1, wobei die Mischung mischbarer Polymere anfänglich eine Barriere gegenüber einer Permeation bzw. Durchdringung durch den aktiven Wirkstoff bereitstellt.

5. System nach Anspruch 4, wobei der aktive Wirkstoff in einer inneren Matrix enthalten ist.

6. System nach Anspruch 5, wobei der aktive Wirkstoff in der inneren Matrix in einer Menge von etwa 0,1 Gew.-% bis etwa 100 Gew.-%, bezogen auf das Gesamtgewicht der inneren Matrix mit dem aktiven Wirkstoff, vorliegt.

7. System nach Anspruch 1, wobei der Unterschied zwischen dem Löslichkeitsparameter des Poly(ethylen-co-(meth)acrylats) und wenigstens einem Löslichkeitsparameter des zweiten Polymers nicht mehr als 5 $J^{1/2}/cm^{3/2}$ beträgt.

8. System nach Anspruch 1, wobei das zweite Polymer ein Polyvinylalkylat-Homopolymer oder ein Kopolymer ist.

9. System nach Anspruch 1, wobei das zweite Polymer ein Polyalkyl- und/oder -aryl-Methacrylat oder ein Acrylat oder ein Kopolymer ist.

10. System nach Anspruch 1, wobei das zweite Polymer ein Polyvinyl-Acetal oder ein Kopolymer ist.

11. System nach Anspruch 1, wobei der aktive Wirkstoff hydrophob ist und ein Molekulargewicht von nicht mehr als 1200 g/mol aufweist.

12. System nach Anspruch 1, wobei das Poly(ethylen-co-(meth)acrylat) in der Mischung mischbarer Polymere in einer Menge von etwa 0,1 Gew.-% bis etwa 99,9 Gew.-%, bezogen auf das Gesamtgewicht der Mischung, vorliegt.

13. System nach Anspruch 1, wobei das zweite Polymer in der Mischung mischbarer Polymere in einer Menge von etwa 0,1 Gew.-% bis etwa 99,9 Gew.-%, bezogen auf das Gesamtgewicht der Mischung, vorliegt.

**14.** System nach Anspruch 1, welches in Form von Mikrosphären bzw. -kugeln, Perlen, Stäben bzw. Stäbchen, Fasern oder anderen geformten Objekten vorliegt.

**15.** System nach Anspruch 14, wobei die kritische Dimension des Objekts nicht mehr als etwa 10.000 Mikron beträgt.

**16.** System nach Anspruch 1, welches in Form eines Films vorliegt.

**17.** System nach Anspruch 16, wobei die Dicke des Films nicht mehr als etwa 1000 Mikron beträgt.

**18.** System nach Anspruch 16, wobei der Film ein Feld bzw. Pflaster oder eine Beschichtung auf einer Oberfläche bildet.

**19.** Abgabesystem für einen aktiven Wirkstoff nach Anspruch 1, wobei eine Abgabe des aktiven Wirkstoffs vornehmlich unter einer Permeationssteuerung stattfindet.

**20.** Abgabesystem für einen aktiven Wirkstoff nach Anspruch 1, wobei:

der aktive Wirkstoff hydrophob ist und ein Molekulargewicht von nicht mehr als 1200 g/mol aufweist;
der Unterschied zwischen dem Löslichkeitsparameter des aktiven Wirkstoffs und dem Löslichkeitsparameter des Poly(ethylen-co-(meth)acrylats) nicht mehr als etwa 10 $J^{l/2}/cm^{3/2}$ beträgt und der Unterschied zwischen dem Löslichkeitsparameter des aktiven Wirkstoffs und wenigstens einem Löslichkeitsparameter des zweiten Polymers nicht mehr als 10 $J^{1/2}/cm^{3/2}$ beträgt; und
der Unterschied zwischen dem Löslichkeitsparameter des Poly(ethylen-co-(meth)acrylats) und wenigstens einem Löslichkeitsparameter des zweiten Polymers nicht mehr als 5 $J^{1/2}/cm^{3/2}$ beträgt.

**21.** Medizinische Vorrichtung mit dem Abgabesystem für einen aktiven Wirkstoff nach Anspruch 1.

**22.** Medizinische Vorrichtung mit dem Abgabesystem für einen aktiven Wirkstoff nach Anspruch 19.

**23.** Medizinische Vorrichtung, mit dem Abgabesystem für einen aktiven Wirkstoff nach Anspruch 20.

**24.** Medizinische Vorrichtung mit:

einer Substratoberfläche;
einer polymeren Grundierungsschicht, die an der Substratoberfläche befestigt ist; und
einer polymeren Oberflächenbeschichtungsschicht, die an der polymeren Grundierungsschicht befestigt ist;
wobei die polymere Oberflächenbeschichtungsschicht das Abgabesystem für einen aktiven Wirkstoff nach Anspruch 1 oder 11 aufweist, in dem der aktive Wirkstoff in der Mischung mischbarer Polymere integriert ist.

**25.** Medizinische Vorrichtung nach Anspruch 24, wobei die polymere Grundierungsschicht ein Polyurethan aufweist.

**26.** Medizinische Vorrichtung nach Anspruch 24, welche eine implantierbare Vorrichtung darstellt.

**27.** Medizinische Vorrichtung nach Anspruch 24, welche eine extrakorporale Vorrichtung darstellt.

**28.** Medizinische Vorrichtung nach Anspruch 24, die aus der Gruppe ausgewählt ist, die aus einem Stent, einem Stent-Transplantat, einem Anastomoseverbinder, einer Leitung, einer Nadel, einem Führungsdraht, einem Katheter, einem Sensor, einem chirurgischen Instrument, einem Angioplastieballon, einer Wunddrainage, einem Shunt, einem Rohr, einem Urether-Insert, einem Pellet, einem Implantat, einem Blutoxygenator, einer Pumpe, einem Gefäß-Transplantat, einem Ventil, einem Herzschrittmacher, einer orthopädischen Vorrichtung, einer Ersatzvorrichtung für Nuclei pulposi und einer intraokularen Linse besteht.

**29.** Medizinische Vorrichtung nach Anspruch 24, wobei die Vorrichtung ein Stent ist.

**30.** Medizinische Vorrichtung nach Anspruch 24 oder 29, wobei eine Abgabe des aktiven Wirkstoffs vornehmlich unter Permeationssteuerung erfolgt.

**31.** Verfahren zur Ausbildung eines Abgabesystems für einen aktiven Wirkstoff nach Anspruch 1, das beinhaltet:

Kombinieren eines Poly(ethylen-co-(meth)acrylats) und eines zweiten Polymers wie in Anspruch 1 definiert, um eine Mischung mischbarer Polymere auszubilden; und
Kombinieren eines aktiven Wirkstoffs mit der Mischung mischbarer Polymere.

**32.** Verfahren nach Anspruch 31, wobei der aktive Wirkstoff in der Mischung mischbarer Polymere integriert bzw. enthalten ist.

**33.** Verfahren nach Anspruch 31, wobei der aktive Wirkstoff in einer inneren Matrix integriert ist und die Mischung mischbarer Polymere zunächst eine Barriere gegenüber einer Permeation durch den aktiven Wirkstoff bereitstellt.

**34.** Verfahren nach Anspruch 31, wobei der aktive Wirkstoff hydrophob ist und ein Molekulargewicht von nicht mehr als etwa 1200 g/mol aufweist.

## Revendications

**1.** Système d'administration d'agent actif comportant un agent actif et un mélange de polymères miscibles comportant un poly(éthylène-co-(méth)acrylate) et un second polymère n'incluant pas de poly(éthylène-acétate de vinyle), dans lequel le second polymère est un homopolymère ou un copolymère d'alkylat polyvinylique, un poly(alkyle) et/ou un méthacrylate d'aryle ou un acrylate ou un copolymère, un polyacétal de vinyle ou un copolymère ou un alkyléther polyvinylique ou un copolymère ; et dans lequel :

chaque élément parmi l'agent actif, le poly(éthylène-co-(méth)acrylate) et le second polymère a un paramètre de solubilité ; et au moins une des relations suivantes est vraie :
la différence entre le paramètre de solubilité de l'agent actif et le paramètre de solubilité du poly(éthylène-co-(méth)acrylate) n'est pas supérieure à environ 10 $J^{1/2}cm^{3/2}$; et
la différence entre le paramètre de solubilité de l'agent actif et au moins un paramètre de solubilité du second polymère n'est pas supérieure à environ 10 $J^{1/2}cm^{3/2}$.

**2.** Système selon la revendication 1, dans lequel l'agent actif est incorporé dans le mélange de polymères miscibles.

**3.** Système selon la revendication 2, dans lequel l'agent actif est présent dans le mélange de polymères miscibles dans une quantité d'environ 0,1 % en poids à environ 80 % en poids, sur la base du poids total du mélange de polymères miscibles et de l'agent actif.

**4.** Système selon la revendication 1, dans lequel le mélange de polymères miscibles produit initialement une barrière à la perméation de l'agent actif.

**5.** Système selon la revendication 4, dans lequel l'agent actif est incorporé dans une matrice interne.

**6.** Système selon la revendication 5, dans lequel l'agent actif est présent dans la matrice interne dans une quantité d'environ 0,1 % en poids à environ 100 % en poids, sur la base du poids total de la matrice interne incluant l'agent actif.

**7.** Système selon la revendication 1, dans lequel la différence entre le paramètre de solubilité du poly(éthylène-co-(méth)acrylate) et au moins un paramètre de solubilité du second polymère n'est pas supérieure à environ 5 $J^{1/2}cm^{3/2}$.

**8.** Système selon la revendication 1, dans lequel le second polymère est un homopolymère ou un copolymère d'alkylat polyvinylique.

**9.** Système selon la revendication 1, dans lequel le second polymère est un polyalkyle et/ou un méthacrylate d'aryle ou un acrylate ou un copolymère.

**10.** Système selon la revendication 1, dans lequel le second polymère est un polyacétal de vinyle ou un copolymère.

**11.** Système selon la revendication 1, dans lequel l'agent actif est hydrophobe et a un poids moléculaire non supérieur à environ 1 200 g/mole.

**12.** Système selon la revendication 1, dans lequel le poly(éthylène-co-(méth)acrylate) est présent dans le mélange de polymères miscibles dans une quantité d'environ 0,1 % en poids à environ 99,9 % en poids, sur la base du poids total du mélange.

**13.** Système selon la revendication 1, dans lequel le second polymère est présent dans le mélange de polymères miscibles dans une quantité d'environ 0,1 % à environ 99,9 %, sur la base du poids total du mélange.

**14.** Système selon la revendication 1 qui se présente sous la forme de microsphères, de billes, de tiges, de fibres ou d'autres objets mis en forme.

**15.** Système selon la revendication 14, dans lequel la dimension critique de l'objet n'est pas supérieure à environ 10 000 microns.

**16.** Système selon la revendication 1 qui se présente sous la forme d'un film.

**17.** Système selon la revendication 16, dans lequel l'épaisseur du film n'est pas supérieure à environ 1 000 microns.

**18.** Système selon la revendication 16, dans lequel le film forme un timbre ou un revêtement sur une surface.

**19.** Système d'administration d'agent actif selon la revendication 1, dans lequel l'administration de l'agent actif s'effectue principalement sous contrôle de la perméation.

**20.** Système d'administration d'agent actif selon la revendication 1, dans lequel :

l'agent actif est hydrophobe et a un poids moléculaire non supérieur à environ 1 200 g/mole ;
la différence entre le paramètre de solubilité de l'agent actif et le paramètre de solubilité du poly(éthylène-co-(méth)acrylate) n'est pas supérieure à environ 10 $J^{1/2}cm^{3/2}$, et la différence entre le paramètre de solubilité de l'agent actif et au moins un paramètre de solubilité du second polymère n'est pas supérieure à environ 10 $J^{1/2}cm^{3/2}$ ; et
la différence entre le paramètre de solubilité du poly(éthylène-co-(méth)acrylate) et au moins un paramètre de solubilité du second polymère n'est pas supérieure à environ 5 $J^{1/2}cm^{3/2}$.

**21.** Dispositif médical comportant le système d'administration d'agent actif de la revendication 1.

**22.** Dispositif médical comportant le système d'administration d'agent actif de la revendication 19.

**23.** Dispositif médical comportant le système d'administration d'agent actif de la revendication 20.

**24.** Dispositif médical comportant :

une surface de substrat ;
une sous-couche polymérique adhérant à la surface de substrat ; et
une couche de finition polymérique adhérant à la sous-couche polymérique ;
dans lequel la couche de finition polymérique comporte le système d'administration d'agent actif de la revendication 1 ou 11 dans lequel l'agent actif est incorporé dans le mélange de polymères miscibles.

**25.** Dispositif médical selon la revendication 24, dans lequel la sous-couche polymérique comporte un polyuréthanne.

**26.** Dispositif médical selon la revendication 24, lequel est un dispositif implantable.

**27.** Dispositif médical selon la revendication 24, lequel est un dispositif extracorporel.

**28.** Dispositif médical selon la revendication 24 choisi parmi le groupe constitué d'une endoprothèse, d'un greffon d'endoprothèse, d'un raccord anastomique, d'un fil, d'une aiguille, un fil-guide, d'un cathéter, d'un capteur, d'un instrument chirurgical, d'un ballonnet d'angioplastie, d'un drain de plaie, d'une dérivation, d'un tube, d'un insert urétral, d'un pellet, d'un implant, d'un oxygénateur sanguin, d'une pompe, d'un greffon vasculaire, d'une valvule, d'un stimulateur cardiaque, d'un dispositif orthopédique, d'un dispositif de remplacement de noyau pulpeux, et d'une lentille intraoculaire.

**29.** Dispositif médical selon la revendication 24, ledit dispositif étant une endoprothèse.

**30.** Dispositif médical selon la revendication 24 ou la revendication 29, dans lequel l'administration de l'agent actif s'effectue principalement sous contrôle de la perméation.

**31.** Procédé pour former un système d'administration d'agent actif tel que revendiqué dans la revendication 1, comportant les étapes consistant à :

combiner un poly(éthylène-co-(méth)acrylate) et un second polymère comme défini dans la revendication 1 pour former un mélange de polymères miscibles ; et
combiner un agent actif avec le mélange de polymères miscibles.

**32.** Procédé selon la revendication 31, dans lequel l'agent actif est incorporé dans le mélange de polymères miscibles.

**33.** Procédé selon la revendication 31, dans lequel l'agent actif est incorporé dans une matrice interne et le mélange de polymères miscibles produit initialement une barrière à la perméation de l'agent actif.

**34.** Procédé selon la revendication 31, dans lequel l'agent actif est hydrophobe et a un poids moléculaire non supérieur à environ 1 200 g /mole.

*Fig. 1*

*Fig. 2*

*Fig. 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20040039437 A

### Non-patent literature cited in the description

- Controlled Drug Delivery: Challenges and Strategies. American Chemical Society, 1997
- **D.W. van Krevelen.** Properties of Polymers. Elsevier
- **D.W. van Krevelen.** Properties of Polymers. Elsevier, 1990
- **M. J. He ; W. X. Chen ; X. X. Dong.** Polymer Physics. FuDan University Press, 2000